Europäisches Patentamt

European Patent Office

Office européen des brevets

(11) Numéro de publication: **0 237 398 B1**

(12) **FASCICULE DE BREVET EUROPEEN**

(45) Date de publication de fascicule du brevet: **01.07.92**

(51) Int. Cl.5: **A61K 37/18**, C07K 3/10, C07K 15/04, C07K 15/06, C12P 21/06, A61K 7/48, A61K 9/50

(21) Numéro de dépôt: **87400410.4**

(22) Date de dépôt: **24.02.87**

(54) Utilisation de polypeptides biologiquement actifs et compositions les contenants.

(30) Priorité: **25.02.86 FR 8602576**

(43) Date de publication de la demande:
**16.09.87 Bulletin 87/38**

(45) Mention de la délivrance du brevet:
**01.07.92 Bulletin 92/27**

(84) Etats contractants désignés:
**BE CH DE FR GB IT LI LU NL**

(56) Documents cités:
**EP-A- 0 049 666       EP-A- 0 101 063**
**EP-A- 0 169 115       EP-A- 0 170 550**
**FR-A- 2 321 849       US-A- 3 558 770**

(73) Titulaire: **LVMH RECHERCHE**
**48-50, rue de Seine**
**F-92703 Colombes Cédex(FR)**

(72) Inventeur: **Redziniak, Gérard**
**101, rue des Fauvettes**
**F-45590 Saint-Cyr-en-Val(FR)**
Inventeur: **Meybeck, Alain**
**20 ter, rue de Bezons**
**F-92400 Courbevoie(FR)**

(74) Mandataire: **Warcoin, Jacques et al**
**Cabinet Régimbeau 26, avenue Kléber**
**F-75116 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne l'utilisation de polypeptides pour la préparation de compositions cosmétiques, de compositions dermatologiques ou de compositions utiles pour leur action sur les cellules de la peau in vitro, les compositions à usage non thérapeutique obtenues et l'utilisation de polypeptides pour la préparation de compositions dermatologiques.

Les extraits d'organes ou de tissus d'animaux ou de microorganismes sont connus depuis longtemps pour leur pouvoir de régénération de la peau et sont utilisés en cosmétologie et en pharmacie dermatologique.

Toutefois, ces extraits sont souvent préparés par des techniques conduisant à des produits difficilement reproductibles et de composition variable, en particulier lorsque ces procédés impliquent des thermodégradations comme cela est décrit, par exemple, dans le brevet français n° 2 472 385.

Ces variations dans la nature et la structure des composants des extraits ainsi préparés influent, bien entendu, sur leurs activités qui sont, elles aussi, difficilement reproductibles.

Par ailleurs, l'activité de nombreux extraits, obtenus par des procédés connus à partir d'organes ou de tissus d'animaux ou de microorganismes sur la régénération de la peau est limitée en raison de la présence dans ces extraits de quantité plus ou moins importante de substances possédant une action mitostatique, voire toxique, vis-à-vis des cellules de la peau.

En outre, par les documents EP-A-49 666, EP-A-169 115, EP-A-170 550 on connaît des peptides biologiquement actifs obtenus en traitant des substances naturelles telles que la caséine ou le fibrinogène avec un agent d'hydrolyse comprenant la chymotrypsine notamment. Ces polypeptides sont actifs en tant que produits biologiques.

Les études poursuivies par la Demanderesse, lui ont permis de mettre au point la possibilité d'obtenir des polypeptides possédant des propriétés particulièrement utiles, notamment en dermatologie, en cosmétique et pour les cultures de cellules, tout en évitant des effets mitostatiques ou cytotoxiques.

En effet, la présente invention concerne l'utilisation précitée, des polypeptides caractérisés en ce qu'ils sont obtenus par hydrolyse enzymatique de substances biologiques naturelles au moyen de l'alpha-chymotrypsine seule ou associée à une autre enzyme protéolytique telle que la trypsine.

Pour la description de la présente invention,on entend par l'expression "substances biologiques naturelles" des macromolécules, généralement des protéines, présentes dans des organismes vivants, par exemple des organes ou tissus animaux, des fluides biologiques ou des microorganismes.

Les essais réalisés par la Demanderesse ont montré que les polypeptides obtenus par le procédé de l'invention possédaient une grande activité biologique, en particulier sur la multiplication des cellules de la peau in vitro, telles que fibroblastes, mélanocytes ou keratinocytes, leur protection contre certaines agressions telles que les rayons ultra-violets, et, in vivo, sur la restructuration de la peau et la prévention de son vieillissement actinique ou naturel.

Ces essais ont montré, en outre, que l'activité biologique de ces polypeptides était, de façon inattendue, généralement et significativement plus importante que celle des produits déjà connus et utilisés pour des applications identiques.

En outre, et de façon tout aussi inattendue des essais réalisés sur cultures de cellules de la peau par la Demanderesse ont montré que les polypeptides ne présentent généralement aucune cytotoxicité, ou une cytotoxicité beaucoup plus faible que celle des extraits connus d'organes, de tissus d'animaux ou de microorganismes dont il a été fait mention plus haut.

De plus, il a été mis en évidence que certains polypeptides correspondant à une gamme de poids moléculaire déterminée étaient plus actifs que ceux ayant des poids moléculaires inférieurs ou supérieurs.

C'est pourquoi la présente invention concerne plus particulièrement l'utilisation des polypeptides de poids moléculaire inférieur à 20 000 Daltons, et plus particulièrement encore des polypeptides de poids moléculaire inférieur à 10 000 Daltons.

Le procédé de préparation de ces polypeptides biologiquement actifs par hydrolyse de substances naturelles en milieux aqueux, est caractérise en ce qu'il comporte les étapes principales suivantes :

a) on prépare un homogénéisat aqueux de substances naturelles,

b) on réalise l'hydrolyse enzymatique avec un agent d'hydrolyse comportant de l'alpha-chymotrypsine,

c) on recueille l'hydrolysat, et

d) on sépare de l'hydrolysat une fraction comportant les polypeptides de poids moléculaire déterminé et on recueille cette fraction.

La préparation de l'homogénéisat aqueux de substances naturelles comprend le broyage des matières premières biologiques, lorsque celles-ci sont solides.

De préférence, selon l'invention, on sépare lors de l'étape d) ci-dessus, la fraction de l'hydrolysat comportant les polypeptides de poids moléculaire inférieur à environ 20 000 Daltons, et en particulier de préférence inférieur à environ 10 000 Daltons.

En outre, on peut également selon l'invention, de l'hydrolysat ou de la fraction d'hydrolysat ci-dessus, éliminer les polypeptides de poids moléculaires inférieurs à environ 1 000 Daltons.

Les matières premières biologiques utilisées pour la mise en oeuvre du procédé selon l'invention sont d'origines variées, comme cela apparaîtra dans les exemples. Elles sont notamment d'origine animale ou issues de microorganismes, et en particulier issues de jeunes animaux ou d'embryons. A titre d'exemples donnés uniquement à titre indicatif et nullement limitatifs, on citera plus particulièrement comme matières :

- des organes ou des tissus animaux : coeur, rate, thymus, peau, cerveau ou foie de bovins, d'ovins ou d'embryons bovins ou de poulets, placenta bovin, ovin ou humain,
- des fluides ou sécrétions biologiques : serum de veau ou de boeuf, lait, colostrum,
- des protéines : protéines de tissus conjonctifs (collagène, élastine, réticuline, fibronectine), protéines fibreuses (fibroïne de la soie, kératine de poils ou de cheveux), séricine,
- des levures : saccharomyces cerevisiae.

Lorsque les substances naturelles mises en oeuvre sont solides, elles sont de préférence broyées avant d'être hydrolysées pour obtenir un hydrolysat aqueux. L'obtention d'un broyat peut être réalisée par des techniques connues, en particulier par broyage mécanique et/ou hydraulique des substances congelées ou non.

L'hydrolyse enzymatique constitue un aspect important du procédé, c'est en effet elle qui permet d'obtenir des polypeptides ayant la caractéristique structurelle évoquée précédemment.

Pour cette hydrolyse, on utilisera comme enzyme l'alpha-chymotrypsine seule ou associée à une autre enzyme protéolytique telle que la trypsine.

Ladite association comprendra de préférence des quantités voisines d'alpha-chymotrypsine et de l'autre enzyme protéolytique.

Cette hydrolyse est mise en oeuvre dans le domaine d'activité optimum de la ou des enzymes, c'est-à-dire en général à une température de l'ordre de 37°C et à un pH maintenu à une valeur proche de 8, le traitement étant effectué de préférence sous agitation douce, en général pendant 1 heure environ.

De façon générale, la quantité d'enzyme ajoutée ne constitue pas un élément caractéristique du procédé car elle peut dépendre de l'activité de la ou des enzymes utilisées, et de la nature de la substance utilisée. A titre d'exemple, dans le cas de l'utilisation d'un mélange alpha-chymotrypsine-trypsine dont l'activité enzymatique est comprise entre 1 000 et 1 500 unités BAEE/mg de solide pour la trypsine et entre 500 et 1 000 unités ATEE/mg de solide pour l'alpha-chymotrypsine, la quantité d'enzyme utilisée est de l'ordre de 1 à 4 % en poids par rapport au poids d'organe mis en oeuvre, mais plus particulièrement 1 %.

A la fin de cette étape d'hydrolyse, il importe en général d'écarter les éléments solides ou susceptibles de colmater les filtres et de gêner les opérations suivantes. C'est pourquoi, on préfère effectuer une filtration de type filtration sur filtre-presse.

De même, un chauffage modéré peut permettre d'éliminer certaines matières grasses en inactivant en outre les enzymes. Ainsi, à la fin de l'hydrolyse et avant filtration sur filtrepresse, l'hydrolysat brut peut être porté en l'espace de quelques minutes à une température de 80°C à 95°C et y est maintenu pendant 10 à 15 minutes. On laisse ensuite refroidir à température ordinaire. On procède alors comme indiqué plus haut, à la filtration sur filtre presse.

On obtient une solution dont on élimine les polypeptides et autres macromolécules de poids moléculaires supérieurs à une valeur déterminée en utilisant par exemple une membrane de filtration ayant un "seuil de coupure" de 20 000 D ou de 10 000 D ("cut off" 20 000 ou 10 000). On recueille le filtrat obtenu.

Suivant une variante du présent procédé, on soumet ledit filtrat à une filtration supplémentaire pour éliminer les peptides de faible poids moléculaire, en particulier, on effectue une filtration au moyen d'une membrane à "seuil de coupure" à 1000 D et on conserve le retentat.

Bien entendu, il est possibile d'utiliser d'autres procédés de séparation qui permettent de sélectionner des poids moléculaires.

Enfin, les polypeptides obtenus par le procédé peuvent être stérilisés, par exemple par autoclavage, par filtration stérilisante ou par tyndallisation. Les produits peuvent également être lyophilisés.

Les polypeptides sont utiles pour leur action sur les cellules.

C'est pourquoi, la présente invention concerne les compositions cosmétiques et les compositions utiles pour leurs actions sur les cellules de la peau in vitro, caractérisées en ce qu'elles comportent une quantité biologiquement active de polypeptides définis précédemment et un support acceptable.

Elle concerne également l'utilisation des polypeptides tels que définis précédemment avec un support acceptable pour l'obtention d'une composition dermatologique.

La présente invention concerne plus particulièrement les compositions cosmétiques, les compositions à

3

EP 0 237 398 B1

usage topique externe non thérapeutiques, les utilisations dermatologiques, et les compositions destinées à la mise en oeuvre de cultures cellulaires.

En effet, ces polypeptides agissent au niveau des cellules de la peau telles que fibroblastes, mélanocytes ou kératinocytes, en particulier au niveau de la stimulation du métabolisme cellulaire et de l'activation de la réplication des cellules, ce qui les rend intéressantes par exemple dans l'activation de la cicatrisation cutanée, dans la prévention du vieillissement et dans la réparation des dommages causés aux cellules, en particulier par les rayonnements ultra-violets, et en général en cosmétologie et en dermatologie.

Les propriétés des polypeptides définis précédemment peuvent également être mises à profit dans les milieux de culture de cellules animales. Ces polypeptides favorisent la croissance des cellules et peuvent remplacer le sérum de veau en tout ou partie.

En outre, l'activité des polypeptides peut être renforcée en associant à d'autres composés biologiquement actifs, par exemple des vitamines telles que vitamine vitamine E ou leurs dérivés, des hydrolysats de protéines tell que l'élastine ou le collagène. La présente invention comprend donc aussi les compositions comportant de telles associations.

D'autre part, les polypeptides, ou lesdites associations peuvent être avantageusement présentés sous des formes particulières permettant d'en développer l'activité. Par exemple, on peut encapsuler ces polypeptides ou associations dans des liposomes. On obtient ainsi des compositions qui sont de façon inattendue significativement plus actives que lesdits polypeptides ou associations à l'état libre.

Les supports sont en particulier des supports cosmétiques ou pharmaceutiques connus de l'homme de métier.

Par exemple, on peut préparer des crèmes oxygénantes pour la peau, des crèmes cicatrisantes, des crèmes régénérantes, des lotions de traitement capillaire des crèmes anti-rides, des lotions après-rasage, des produits solaires, des laits après-soleil réparateurs et des crèmes antiérythèmes ou des laits et crèmes pour le corps. Bien entendu, les compositions selon la présente invention peuvent comporter d'autres principes actifs ayant une activité de même type ou une activité complémentaire.

Les exemples ci-après,donnés seulement à titre indicatif sans être pour autant limitatifs, permettent de faire mieux comprendre les caractéristiques et avantages de la présente invention.

EXEMPLE 1 :

Peptides de thymus de veau (référence Ex 1) obtenus après hydrolyse par l'alpha-chymotrypsine

On hache et broie le plus finement possible, en présence de 4 litres d'eau déminéralisée, 1 kilogramme de thymus de veau fraîchement prélevés dans des conditions aseptiques.

Le tout est homogénéisé après l'addition de 0,6 grammes de chlorure de calcium. Le pH de la suspension obtenue est ajusté à 8 par une solution de soude à 40 %, et on chauffe pour amener la température à 37°C environ. On ajoute alors 0,2 litres d'une solution aqueuse à 5 % en poids d'alpha-chymotrypsine pour provoquer l'hydrolyse des protéines.

L'hydrolyse est poursuivie pendant 3 heures en maintenant la température à 37°C et le pH à 8 sous agitation douce, puis on chauffe à 80°C pendant 15 minutes. On laisse refroidir à température ordinaire (23°C environ).

La suspension obtenue est d'abord filtrée sur filtrepresse, puis le filtrat est ultra-filtré sur membrane "cut off" 10 000. L'ultra-filtrat est stérilisé à l'autoclave à 110°C pendant 20 minutes. Cette stérilisation n'est pas nécessaire si on a opéré précédemment dans des conditions stériles.

La quantité d'extrait sec dans la solution de peptides obtenue est de 30 g/litre.

EXEMPLE 2 :

Peptides de thymus de veau (référence Ex 2), obtenus après hydrolyse par un mélange alpha-chymotrypsine-trypsine

On opère comme dans l'exemple 1, à partir d'un kilogramme de thymus de veau, avec comme agent d'hydrolyse 0,2 litres de solution aqueuse à 5 % en poids d'un mélange contenant des quantités voisines d'alpha-chymotrypsine et de trypsine.

La solution finale de peptides a une teneur en extrait sec de 40 g/litre.

EXEMPLE 3 :

4

Peptides de coeur de boeuf (référence Ex 3) obtenus après hydrolyse par un mélange alpha-chymotrypsine-trypsine

On opère comme dans l'exemple 2, à partir d'un kilogramme de coeur de boeuf fraîchement prélevé dans des conditions aseptiques, en utilisant le même mélange d'enzymes.

La solution finale de peptides a une teneur en extrait sec de 60 g/litre.

EXEMPLE 4 :

Peptides de tissu placentaire humain (référence Ex 4) obtenus après hydrolyse par l'alpha-chymotrypsine

On opère comme dans l'exemple 1, à partir d'un kg de placenta humain fraîchement recueilli et débarassé totalement du sang.

La solution finale de peptides a une teneur en extrait sec de 40 g/litre.

EXEMPLES 5 et 6 :

Peptides de tissu placentaire humain (référence Ex 5 et Ex 6) obtenus après hydrolyse par un mélange alpha-chymotrypsine-trypsine

On opère comme dans l'exemple 2, à partir d'un kg de placenta humain fraîchement recueilli et débarassé totalement du sang, on obtient une solution de peptides de poids moléculaire ne dépassant pas 10 000 Daltons, que l'on stérilise à l'autoclave (référence Ex 5).

Sur une partie de la solution ci-dessus, on effectue une ultra-filtration supplémentaire sur membrane "cut off" 1 000. L'ultra-filtrat est éliminé. Le rétentat obtenu, qui sera stérilisé, est une solution de peptides dont les poids moléculaires sont compris entre 1 000 et 10 000 (référence Ex 6).

Le poids d'extrait sec de la solution de peptides avant la dernière ultra-filtration (solution Ex 5) est de 40 grammes par litre, celui du rétentat après cette ultra-filtration (solution Ex 6) est de 30 grammes/litre.

EXEMPLE 7 :

Peptides de sérum de boeuf (référence Ex 7) obtenus après hydrolyse par l'alpha-chymotrypsine

On opère comme dans l'exemple 1 avec 5 litres de sérum de boeuf prélevé dans des conditions aseptiques dont on ajuste le pH à 8 par une solution de soude à 40 %.

La solution finale de peptides a une teneur en extrait sec de 60 grammes par litre.

EXEMPLES 8 à 10

Peptides de sérum de boeuf (références Ex 8, Ex 9, Ex 10) obtenus après hydrolyse par un mélange alpha-chymotrypsine trypsine

On opère comme à l'exemple 7, à partir de 5 litres de sérum de boeuf, prélevés dans des conditions aseptiques avec comme agent d'hydrolyse 0,2 litres d'une solution aqueuse enzymatique identique à celle employée dans l'exemple 2. On obtient une solution de peptides de poids moléculaire ne dépassant pas 10 000 Daltons que l'on stérilise à l'autoclave (référence Ex 8).

Une partie de la solution ci-dessus subit une ultra-filtration supplémentaire sur membrane "cut off" 1 000. L'ultra-filtrat et le rétentat sont stérilisés à l'autoclave. Le premier contient des peptides de poids moléculaire inférieur à 1 000 (référence Ex 9), le second des peptides de p. m. compris entre 1 000 et 10 000 (référence Ex 10).

Les extraits secs pour les solutions obtenues sont respectivement :
- solution Ex 8 : 50 g/litre,
- solution Ex 9 : 10 g/litre,
- solution Ex 10 : 40 g/litre.

EXEMPLE 11 :

Peptides de Saccharomyces cerevisiae (référence Ex 11), obtenus après hydrolyse par un mélange alpha-chymotrypsine-trypsine

Les levures contenues dans 50 ml d'une suspension aqueuse concentrée par centrifugation, sont broyées au moyen d'un homogénéiseur hydraulique en présence de 500 ml d'une solution tampon à pH 7,5. Ce broyat est porté à 37°C, et on effectue alors une hydrolyse enzymatique par addition de 20 ml de la solution aqueuse d'enzymes utilisée à l'exemple 2. La température est maintenue à cette valeur pendant une heure. A la fin de l'hydrolyse, le mélange est ultra-filtré sur membranes "cut off" 10.000.

L'extrait sec de la solution finale de polypeptides est de 30 grammes par litre.

EXEMPLES 12 et 13 :

Peptides de thymus de veau ou de sérum de boeufs encapsulés dans des liposomes (respectivement ref Ex 12 et Ex 13)

La préparation de liposomes est effectuée selon le procédé décrit dans le brevet français n° 2 521 565.

On dissout 90 grammes de lécithine et 10 grammes de cholestérol dans 1 litre de dichlorométhane. La solution obtenue est atomisée dans un courant d'air chaud à 60°C. On obtient 100 grammes d'une poudre lipidique qui est dispersée par agitation mécanique dans 2 litres d'une solution aqueuse contenant 0,15 ‰ d'extrait sec de peptides de thymus de veau obtenus selon l'exemple 2 ou de peptides de sérum de boeuf obtenus selon l'exemple 7. On obtient une suspension de liposomes dont on améliore la stabilité par le procédé décrit dans la demande de brevet français n° 2 534 487, consistant essentiellement à homogénéiser ladite suspension de liposomes dans un homogénéiseur sous pression pendant 15 minutes à 300 bars et à un débit de 50 litres par heure.

On obtient ainsi une suspension homogène de liposomes contenant les peptides de thymus de veau (Ex. 12), ou les peptides de sérum de boeuf (Ex. 13). La taille moyenne de ces liposomes est de 100 nanomètres.

EXEMPLE 14 :

Composition contenant des peptides de coeur de boeuf et des hydrolysats d'élastine et de collagène (ref. Ex 14)

On prépare une solution aqueuse dont la composition est la suivante :

| | |
|---|---|
| - peptides de coeur de boeuf en solution aqueuse à 3 % en poids d'extrait sec (Ex 3) selon l'exemple 3 | 33 ml |
| - hydrolysat d'élastine | 1 g |
| - hydrolysat de collagène | 1 g |
| - Eau distillée qsp | 100 ml |

EXEMPLE 15 :

Activité des peptides selon l'invention sur la multiplication de fibroblastes en culture

Des tests ont été réalisés sur des cultures de fibroblastes dans un milieu M.E.M. (milieu minimum de Eagle - GIBCO® ), en présence ou non de peptides selon l'invention, pour mesurer l'activité multiplicatrice de ces peptides.

Les peptides et compositions de peptides selon l'invention ont été testées. En outre, à titre comparatif, les peptides suivants ont également été testés :
- peptides de sérum de boeuf (référence SBT) obtenus après hydrolyse par la trypsine seule selon le procédé décrit à l'exemple 7 ci-dessus dans lequel on a remplacé l'alpha-chymotrypsine par une quantité de trypsine présentant la même activité enzymatique exprimée en unités BAEE/mg de solide. Extrait sec : 30 mg/ml ;
- peptides de thymus du commerce (référence TPP) préparés par extraction aqueuse d'un broyat de thymus, suivie de l'élimination des substances de haut poids moléculaire .

Le tableau 1 présente les résultats de ces tests.

Les extraits ont été évalués comme très actifs (xxx), actifs (xx), ou moyennement actifs (x).

Par rapport à un extrait du commerce de même origine, les extraits selon l'invention sont plus actifs (Ex1 et TPP).

L'ajout d'alpha-chymotrypsine au système enzymatique permet d'augmenter l'activité (Ex 8-SBT).

L'élimination des polypeptides de faible poids moléculaire ( < 1000 D ) permet d'augmenter l'activité des extraits (Ex 5-Ex 6).

On remarque que l'association de peptides selon l'invention, tels que ceux de coeur de boeuf avec des hydrolysats d'élastine et de collagène, améliore encore l'activité (Ex 14, Ex 3).

Il apparaît donc clairement que les peptides selon l'invention favorisent la régénération cellulaire.

TABLEAU 1

| REFERENCE | ORIGINE DES PEPTIDES | SYSTEME[1] ENZYMATIQUE | POIDS MOLECULAIRE (PM) | ACTIVITE |
|---|---|---|---|---|
| Ex 1 | Thymus de veau | A | < 10 000 | xxx |
| Ex 3 | Coeur de boeuf | B | < 10 000 | x |
| Ex 4 | Placenta humain | A | < 10 000 | xxx |
| Ex 5 | Placenta humain | B | < 10 000 | xx |
| Ex 6 | Placenta humain | B | $10^3 < PM < 10^4$ | xxx |
| Ex 8 | Sérum de boeuf | B | < 10 000 | xx |
| Ex 11 | Saccharomyces Cerevisiae | B | < 10 000 | x |
| Ex 14 | Coeur de boeuf + élastine + collagène | B | < 10 000 | xxx |
| SBT | Sérum de boeuf | C | < 10 000 | x |
| TPP | Extrait de thymus de veau du commerce | | | x |

(1) hydrolyse enzymatique effectuée avec :

A = alpha-chymotrypsine seule
B = mélange équiactif d'alpha-chymotrypsine et de trypsine
C = trypsine seule

EXEMPLE 16 :

Protocole des tests de l'exemple 15

On réalise dans trois groupes numérotés de 1 à 3, de trois boîtes de Pétri des cultures de fibroblastes souscutanés de souris, dans 2 millilitres d'un milieu M.E.M. (Milieu Minimum de Eagle) GIBCO® additionné de 100 microgrammes par millilitre de streptomycine et 100 UI par millilitre de pénicilline. Le nombre de cellules à l'ensemencement est de 50 000 cellules par millilitre.

Dans les boîtes n° 1 et n° 2 on ajoute 5 % en poids de sérum de veau total et dans les boîtes n° 3, on ajoute 2,5 % en poids de sérum de veau total.

Puis on remplace dans les boîtes n° 1 les 2 millilitres de milieu (contenant les 5 % de sérum de veau) par la même quantité de milieu frais MEM additionné de 2,5 % en poids de sérum de veau total et de 1 % en poids d'une solution de peptides à tester.

Dans les boîtes 2 et 3, boîtes témoin, on remplace également les 2 millilitres de milieu par la même quantité de milieu frais MEM additionné respectivement de 5 % et de 2,5 % en poids de sérum de veau total.

Pour tout ces tests, la concentration en polypeptides est d'environ 0,2 g/l.

Toutes les boîtes sont alors placées dans des conditions optimales de culture (37°C ; 5 % en $CO_2$ ; 98 % d'humidité relative).

Après trois jours, on traite les cultures à la trypsine pour décoller les cellules et on dénombre les cellules dans chaque boîte pour évaluer l'activité.

EXEMPLE 17 :

Activité des peptides selon l'invention sur la réparation de fibroblastes irradiées par des rayons ultra-violets

Le test est effectué sur une durée de cinq jours. Le premier jour, on ensemence avec 70 000 fibroblastes souscutanés de souris trois groupes de trois boîtes de Petri, numérotés de 1 à 3, contenant du milieu M.E.M. (GIBCO®) additionné de 2,5 % en poids de sérum de veau. Les boîtes n° 3 contiennent, en outre,1 % en poids de la solution aqueuse des peptides à tester.

Les boîtes n° 1 et n° 2 sont des boîtes témoin.

Les trois groupes de boîtes sont ensuite placés pendant 24 heures à l'étuve à 37 °C sous atmosphère de gaz carbonique.

Le deuxième jour, on rince les boîtes par une solution tampon au phosphate monosodique à 1,8 %, ne contenant ni calcium ni magnésium, puis on expose les boîtes n° 2 et 3 en présence de la solution PBS, à un rayonnement ultra-violet UV de 300 nanomètres de longueur d'onde, et de 0,1 milli-Watt/cm² d'énergie.

La durée d'exposition est de 75 secondes qui, pour les boîtes témoin n° 2 correspond dans ces conditions à la mort de 50 % des cellules. Après cette irradiation, la solution saline est remplacée par du milieu de culture M.E.M. additionné de 2,5 % de sérum de veau, la boîte n° 3 contient, coie la veille la même quantité (1%) de solution de peptides à tester. On remplace également dans les boîtes témoin n° 1, qui n'ont pas été irradiées, la solution de rinçage par du milieu M.E.M. additionné de 2,5 % de sérum de veau. Les trois groupes de boîtes sont à nouveau placés à l'étuve à 37°C, sous atmosphère de gaz carbonique pendant 72 heures.

A l'issue de cette période, le milieu de culture est éliminé. On traite les cultures à la trypsine pour décoller les cellules et on effectue une numération dans chaque boîte. Les activités ont été aussi évaluées : très actif (xxx), actif (xx), moyennement actif (x), non actif (-).

Les résultats sont rapportés au tableau 2 ciaprès.

D'après ces résultats, il apparaît donc clairement que les peptides selon l'invention favorisent la réparation des effets néfastes des UV.

   . L'emploi d'alpha-chymotrypsine pour la préparation des peptides est bénéfique (comparaison avec SBT).

   . L'élimination des polypeptides de faible poids moléculaire (< 1 000 D) permet d'augmenter l'activité des extraits.

9

TABLEAU 2

| Référence | Origine des peptides | Enzyme [1] | PM Dalton | Concentration C g/l | Activité |
|---|---|---|---|---|---|
| Ex 2 | thymus de veau | B | $< 10^4$ | 0,4 | xx |
| Ex 3 | coeur de boeuf | B | " | 0,6 | x |
| Ex 7 | sérum de boeuf | A | " | 0,6 | x |
| Ex 8 | sérum de boeuf | B | " | 0,5 | xx |
| Ex 10 | sérum de boeuf | B | $10^3$ à $10^4$ | 0,4 | xxx |
| EX 14 | coeur de boeuf + élastine + collagène | B | $< 10^4$ | 0,1 | xxx |
| SBT | sérum de boeuf | C | " | 0,3 | - |

(1)   hydrolyse enzymatique effectuée avec :

A = alpha-chymotrypsine seule

B = mélange équiactif d'alpha-chymotrypsine et de trypsine

C = trypsine seule

EXEMPLE 18

| Gel cicatrisant | |
|---|---|
| | % en poids |
| . Solution aqueuse de peptides de sérum de boeuf, selon l'exemple 8 | 10 % |
| . Excipient pour gel de CARBOPOL®, qsp | 100 % |

EXEMPLE 19

10

| Crème de soins | |
|---|---|
| | % en poids |
| . Suspension aqueuse de liposomes encapsulant des peptides de thymus de veau, selon l'exemple 12 | 25 % |
| . Excipient émulsionné huile dans eau, qsp | 100 % |

La crème obtenue, utilisée à raison d'une application quotidienne, améliore l'état de surface de la peau.

EXEMPLE 20

| Gel régénérant | |
|---|---|
| | % en poids |
| . Suspension aqueuse de liposomes encapsulant une composition à base de peptides de coeur de boeuf, selon l'exemple 14 | 20 % |
| . Excipient pour gel de CARBOPOL®, qsp | 100 % |

Le gel obtenu est particulièrement avantageux pour régénérer les peaux prématurément vieillies par les rayons ultra-violets.

EXEMPLE 21

| Lait pour le corps | |
|---|---|
| | % en poids |
| . Solution aqueuse de peptides de Saccharomyces cerevisiae, selon l'exemple 11 | 10 % |
| . Excipient, qsp | 100 % |

EXEMPLE 22

| Crème de nuit | |
|---|---|
| | % en poids |
| . Solution aqueuse de peptides de tissu placentaire, selon l'exemple 4 | 5 % |
| . Excipient émulsionné eau dans l'huile, qsp | 100 % |

EXEMPLE 23

| Composition pour milieux de culture cellulaire | |
|---|---|
| | % en poids |
| . Solution aqueuse d'une composition à base de peptides de coeur de boeuf, selon l'exemple 14 | 5 % |
| . Milieu M.E.M.® (Minimum Eagle Medium) | 95 % |

Cette composition peut être avantageusement utilisée pour la culture de fibroblastes.

EXEMPLE 24

| Lait bronzant | |
|---|---|
| | % en poids |
| . Solution aqueuse de peptides de coeur selon l'exemple 3 | 10 % |
| . Acétate de vitamine E | 1 % |
| . Excipient émulsionné huile dans eau, qsp | 100 % |

**Revendications**

**1.** Utilisation, pour la préparation de compositions cosmétiques, de compositions dermatologiques ou de compositions utiles pour leur action sur les cellules de la peau in vitro, de polypeptides biologiquement actifs susceptibles d'être obtenus par un procédé d'hydrolyse de substances naturelles en milieu aqueux comportant les étapes suivantes :

a) on prépare un homogénéisat aqueux de substances naturelles,

b) on réalise l'hydrolyse enzymatique avec un agent d'hydrolyse comportant de l'alpha-chymotrypsine,

c) on recueille l'hydrolysat, et

d) on sépare de l'hydrolysat une fraction comportant les polypeptides de poids moléculaire inférieur à environ 20 000 Daltons et on recueille cette fraction.

**2.** Utilisation selon la revendication 1, selon laquelle l'agent d'hydrolyse de l'étape b) est constitué seulement par l'alpha-chymotrypsine.

**3.** Utilisation selon la revendication 1, selon laquelle l'agent d'hydrolyse est constitué d'alpha-chymotrypsine et de trypsine.

**4.** Utilisation selon l'une des revendications 1 ou 2, selon laquelle à l'étape d) on sépare la fraction comportant les polypeptides de poids moléculaire inférieur à environ 10 000 Daltons.

**5.** Utilisation selon l'une des revendications 1 à 4, selon laquelle à l'étape d) on sépare de l'hydrolysat une fraction comportant les polypeptides de poids moléculaire inférieur à environ 20 000 Daltons et supérieur à environ 1000 Daltons.

**6.** Utilisation selon l'une des revendications 1 à 5, caractérisée en ce qu'en outre, avant de séparer de l'hydrolysat une fraction comportant les polypeptides de poids moléculaire déterminé, on effectue une filtration pour éliminer les particules et agglomérats solides.

**7.** Utilisation selon l'une des revendications 1 à 6, caractérisée en ce les substances naturelles sont issues de matières biologiques d'origine animale ou de microorganismes.

**8.** Utilisation selon l'une des revendications 1 à 7, caractérisée en ce que l'homogénéisat de substances naturelles est préparé à partir des matières premières choisies parmi :

- des organes ou des tissus animaux tels que coeur, thymus, cerveau ou foie de bovins ou d'ovins ou d'embryons bovins ou de poulets, les placentas bovins, ovins ou humains,

- ou des protéines telles que les protéines de tissus conjonctifs comme le collagène, l'élastine la réticuline ou la fibronectine, les protéines fibreuses comme les fibroînes de la soie, la kératine des poils ou des cheveux, ou la séricine,

- ou des levures.

**9.** Compositions cosmétiques et compositions utiles pour leur action sur les cellules de la peau in vitro, caractérisées en ce qu'elles comportent des polypeptides tels que définis selon l'une des revendications 1 à 8, et un support acceptable.

12

**10.** Compositions à usage topique externe selon la revendication 9.

**11.** Compositions selon l'une des revendications 9 ou 10, caractérisées en ce qu'elles comportent en outre du sérum de veau.

**12.** Compositions selon l'une des revendications 9 à 11, caractérisées en ce qu'elles comportent en outre une vitamine ou l'un de ses dérivés tels que l'acétate de vitamine E.

**13.** Compositions selon l'une des revendications 9 à 12, caractérisées en ce qu'elles comportent en outre un hydrolysat de protéine, notamment un hydrolysat d'élastine ou de collagène.

**14.** Compositions selon l'une des revendications 9 à 13, caractérisées en ce que les polypeptides sont inclus dans des liposomes.

**15.** Utilisation des polypeptides tels que définis selon l'une des revendications 1 à 8, avec un support acceptable pour l'obtention d'une composition dermatologique utile pour son action sur les cellules de la peau.

**16.** Utilisation selon la revendication 15, caractérisée en ce que la composition dermatologique est à usage topique externe.

**17.** Utilisation selon l'une des revendications 15 ou 16, caractérisée en ce que la composition comporte en outre du sérum de veau.

**18.** Utilisation selon l'une des revendications 15 à 17, caractérisée en ce que la composition comporte en outre une vitamine ou l'un de ses dérivés tels que l'acétate de vitamine E.

**19.** Utilisation selon l'une des revendications 15 à 18, caractérisée en ce que la composition comporte en outre un hydrolysat de protéine, notamment un hydrolysat d'élastine ou de collagène.

**20.** Utilisation selon l'une des revendications 15 à 19, caractérisée en ce que les polypeptides sont inclus dans des liposomes.

**Claims**

**1.** Use, for the preparation of cosmetic, dermatologic compositions or composition useful for their action on cells, in vitro, of biologically active polypeptides liable to be obtained by hydrolysis of natural substances in aqueous medium, comprising the following steps :
a) preparing an aqueous homogenizate of natural substances,
b) hydrolyzing the product enzymatically with a hydrolysis agent comprising alpha-chymotrypsin,
c) collecting the hydrolyzate, and
d) separating from the hydrolyzate a fraction comprising the polypeptides of particular molecular weight and collecting this fraction.

**2.** Use according to Claim 1, wherein the hydrolysis agent of step b) is constituted only by alpha-chymotrypsin .

**3.** Use according to Claim 1, wherein the hydrolysis agent is constituted by alpha-chymotrypsin and trypsin .

**4.** Use according to Claim 1 or 2, wherein at step d) the fraction comprising the polypeptides of molecular weight less than about 10,000 Daltons is separated.

**5.** Use according to any of claims 1 to 4, according to which at step d), from the hydrolyzate, a fraction comprising the polypeptides of molecular weight less than about 20 000 Daltons and higher than about 1 000 Daltons is separated.

**6.** Use according to any of claims 1 to 5, wherein in addition, before separating from the hydrolyzate the fraction comprising the polypeptides of particular molecular weight, a filtration is carried out to remove the solid particles and agglomerates.

7. Process according to any of claims 1 to 6, wherein the natural substances come from biological materials of animal origin or from microorganisms.

8. Use according to any of the claims 1 to 7, wherein the homogenizate of natural substances is prepared from raw materials selected from among :
   - organs or animal tissues such as : heart, spleen, thymus, skin, brain or liver from bovine or ovine cattle or from bovine embryos or from chickens, bovine, ovine or human placentas,
   - or from biological fluids or secretions such as veal or beef serum, milk, colostrum,
   - or from proteins such as proteins of conjunctive tissues such as collagen, elastin, reticulin, or fibronectin, fibrous proteins like fibroins from silk, keratin from fur or from hair, or sericin,
   - or from yeasts.

9. Cosmetic compositions and compositions useful far their action on cells in vitro, which comprise polypeptides according to any of the claims 1 to 8 and an acceptable support.

10. Compositions for external topical use according to claim 9.

11. Compositions according to claims 9 or 10, which comprise in addition veal serum.

12. Compositions according to any of the claims 9 to 11, which comprise, in addition, a vitamin or one of its derivatives such as vitamin E acetate.

13. Compositions according to any of the claims 9 to 12, which comprise in addition a protein hydrolyzate, particularly a hydrolyzate of elastin and/or collagen.

14. Compositions according to any of the claims 9 to 13, wherein the polypeptides are included in liposomes.

15. Use of polypeptides according to any of the claims 1 to 8, with an acceptable support for the preparation of dermatologic composition useful for its action on cells.

16. Use according to the claim 15, wherein the dermatologic composition is for external topical use.

17. Use according to the claims 15 or 16, wherein the composition comprises in addition veal serum.

18. Use according to any of the claims 15 to 17, wherein the composition comprises in addition a vitamin or one of its derivatives such as vitamin E acetate.

19. Use according to any of the claims 15 to 18, wherein the composition comprises in addition a protein hydrolyzate, particularly a hydrolyzate of elastin and/or collagen.

20. Use according to any of the claims 15 to 19, wherein the polypeptides are included in liposomes.

**Patentansprüche**

1. Verwendung zur Herstellung kosmetischer Zusammensetzungen, dermatologischer Zusammensetzungen oder von Zusammensetzungen, welche wegen ihrer Wirkung in vitro auf die Hautzellen nützlich sind, von biologisch aktiven Polypeptiden, welche durch ein Verfahren zur Hydrolyse von Naturstoffen in wäßrigem Milieu erhalten werden können, umfassend die folgenden Schritte:
   a) man stellt ein wäßriges Homogenisat aus Naturstoffen her,
   b) man führt die enzymatische Hydrolyse mit einem Hydrolysemittel durch, welches $\alpha$-Chymotrypsin umfaßt,
   c) man fängt das Hydrolysat auf und
   d) man trennt aus dem Hydrolysat eine Fraktion ab, welche die Polypeptide mit einem Molekulargewicht von weniger als etwa 20 000 Daltons umfaßt, und fängt diese Fraktion auf.

14

**2.** Verwendung gemäß Anspruch 1, wonach das Hydrolysemittel von Schritt b) nur aus α-Chymotrypsin besteht.

**3.** Verwendung gemäß Anspruch 1, wonach das Hydrolysemittel aus α-Chymotrypsin und Trypsin besteht.

**4.** Verwendung gemäß einem der Ansprüche 1 und 2, wonach man in Schritt d) die Fraktion abtrennt, welche die Polypeptide mit einem Molekulargewicht von weniger als etwa 10 000 Daltons umfaßt.

**5.** Verwendung gemäß einem der Ansprüche 1 bis 4, wonach man in Schritt d) aus dem Hydrolysat eine Fraktion abtrennt, welche die Polypeptide mit einem Molekulargewicht von weniger als etwa 20 000 Daltons und mehr als etwa 1000 Daltons umfaßt.

**6.** Verwendung gemäß einem der Ansprüche 1 bis 5, dadurch gekennzeichnet, daß man darüberhinaus vor dem Abtrennen einer die Polypeptide mit einem bestimmten Molekulargewicht umfassenden Fraktion aus dem Hydrolysat eine Filtration durchführt, um feste Teilchen und Agglomerate zu entfernen.

**7.** Verwendung gemäß einem der Ansprüche 1 bis 6, dadurch gekennzeichnet, daß die Naturstoffe von biologischen Materialien tierischen Ursprungs oder von Mikroorganismen stammen.

**8.** Verwendung gemäß einem der Ansprüche 1 bis 7, dadurch gekennzeichnet, daß das Homogenisat aus Naturstoffen aus Ausgangsmaterialien hergestellt ist, welche aus
   - tierischen Organen oder Geweben wie Herz, Thymusdrüse, Gehirn oder Leber von Rindern oder Schafen oder Rinder- oder Hühnerembryonen, Rinder-, Schaf- oder Humanplazenta,
   - oder Proteinen wie den Bindegewebsproteinen wie Kollegen, Elastin, Reticulin oder Fibronectin, Faserproteinen wie den Seidenfibroinen, Keratin von Tier- oder Menschenhaaren, oder Sericin,
   - oder Hefen ausgewählt sind.

**9.** Kosmetische Zusammensetzungen oder Zusammensetzungen, welche wegen ihrer Wirkung in vitro auf die Hautzellen nützlich sind, dadurch gekennzeichnet, daß sie gemäß einem der Ansprüche 1 bis 8 definierte Polypeptide und einen annehmbaren Träger enthalten.

**10.** Zusammensetzungen zum äußeren topischen Gebrauch gemäß Anspruch 9.

**11.** Zusammensetzungen gemäß einem der Ansprüche 9 oder 10, dadurch gekennzeichnet, daß sie außerdem Kälberserum umfassen.

**12.** Zusammensetzungen gemäß einem der Ansprüche 9 bis 11, dadurch gekennzeichnet, daß sie außerdem ein Vitamin oder eines seiner Derivate wie Vitamin E-acetat umfassen.

**13.** Zusammensetzungen gemäß einem der Ansprüche 9 bis 12, dadurch gekennzeichnet, daß sie außerdem ein Proteinhydrolysat, insbesondere ein Elastin- oder Kollagenhydrolysat, enthalten.

**14.** Zusammensetzungen gemäß einem der Ansprüche 9 bis 13, dadurch gekennzeichnet, daß die Polypeptide in Liposomen eingeschlossen sind.

**15.** Verwendung von gemäß einem der Ansprüche 1 bis 8 definierten Polypeptiden mit einem zur Herstellung einer dermatologischen Zusammensetzung, welche wegen ihrer Wirkung in vitro auf die Hautzellen nützlich ist, annehmbaren Träger.

**16.** Verwendung gemäß Anspruch 15, dadurch gekennzeichnet, daß die dermatologische Zusammensetzung zum topischen äußeren Gebrauch bestimmt ist.

**17.** Verwendung gemäß einem der Ansprüche 15 oder 16, dadurch gekennzeichnet, daß die Zusammensetzung außerdem Kälberserum enthält.

**18.** Verwendung gemäß einem der Ansprüche 15 bis 17, dadurch gekennzeichnet, daß die Zusammensetzung außerdem ein Vitamin oder eines seiner Derivate wie Vitamin E-acetat enthält.

19. Verwendung gemäß einem der Ansprüche 15 bis 18, dadurch gekennzeichnet, daß die Zusammensetzung außerdem ein Proteinhydrolysat, insbesondere ein Elastin- oder Kollagenhydrolysat, enthält.

20. Verwendung gemäß einem der Ansprüche 15 bis 19, dadurch gekennzeichnet, daß die Polypeptide in Liposomen eingeschlossen sind.